# EUROPÄISCHE PATENTSCHRIFT

(11) **EP 2 451 446 B1**
(45) Veröffentlichungstag und Bekanntmachung des Hinweises auf die Patenterteilung: **05.03.2014**
(21) Anmeldenummer: 10731473.4
(22) Anmeldetag: 07.07.2010
(51) Int. Cl.: A61K 9/20

(54) **ZUSAMMENSETZUNG FÜR DIE TABLETTENHERSTELLUNG UND VERFAHREN ZU DEREN HERSTELLUNG**
COMPOSITION FOR THE PRODUCTION OF TABLETS, AND METHOD FOR THE PRODUCTION OF SAID COMPOSITION
COMPOSITION POUR LA FABRICATION DE COMPRIMÉS ET SON PROCÉDÉ DE PRÉPARATION

(30) Priorität: 10.07.2009 EP 09009065
(43) Veröffentlichungstag der Anmeldung: 16.05.2012
(73) Patentinhaber: Merck Patent GmbH, 64293 Darmstadt (DE)
(72) Erfinder: EASSON, James, 64289 Darmstadt (DE); HAMM, Walter, 64331 Weiterstadt (DE); MODDELMOG, Guenter, 64354 Reinheim (DE)
(86) Internationale Anmeldenummer: PCT/EP2010/004135
(87) Internationale Veröffentlichungsnummer: WO 2011/003603

(56) Entgegenhaltungen:
- WO-A1-2008/024044
- US-A1- 2004 162 333
- US-A1- 2009 087 485

## Beschreibung

Die vorliegende Erfindung betrifft ein Verfahren zur Herstellung einer Zusammensetzung für die Tablettenherstellung und eine hierdurch erhaltene Zusammensetzung gemäß Anspruch 1. Bei dieser Zusammensetzung handelt es sich um eine direkt verpressbare Zusammensetzung, die sowohl zu verbesserten Tablettier- als auch zu verbesserten Tabletteneigenschaften führt.

Die Direktverpessung (DC) ist ein einfaches, schnelles, kostengünstiges, Wirkstoff schonendes und flexibles Verfahren in der Tablettenherstellung. Aus verschiedenen Gründen eignen sich jedoch nicht alle zur Formulierung von Tabletten einsetzbaren Komponenten für den Einsatz in diesem Prozess.

Beispielsweise kann pulverförmiges Calciumhydrogenphosphat Dihydrat als Tablettenträgerstoff wegen schlechter Fließeigenschaften und fehlender Kompressibilität meistens nicht ohne spezielle Zusätze oder Behandlungen in der Direkttablettierung eingesetzt werden.

Jedoch ist die Direktverpessung in der Tablettenherstellung einerseits aus Kostengründen üblich. Sie wird andererseits aber auch angewandt zur Formulierung von Preßlingen aus Calciumhydrogenphosphat Dihydrat, um Inkompatibilitäten von Komponenten in den Rezepturen zu vermeiden.

In der Regel sind jedoch nur speziell physikalisch modifizierte Calciumhydrogenphosphat Dihydrate für die Verwendung in diesem Prozess geeignet. Aufgrund des spröden Materialcharakters ist die Kompressibilität dieser Materialien in vielen Rezepturen häufig jedoch nicht ausreichend. Die Zerfallszeiten sind zudem bei den aus diesen DC-Calciumhydrogenphosphat Dihydraten hergestellten Preßlinge, u. a. bedingt durch die geringe Löslichkeit des Calciumhydrogenphospat Dihydrats in wässrigen Medien, teilweise unbefriedigend. Auch organoleptisch weisen DC-Calciumhydrogenphosphat Dihydrate aufgrund der sandigen, scharfkantigen Partikelstruktur und der schlechten Löslichkeit Nachteile auf, sodass ihre Verwendung in oral zerfallenden Darreichungsformen eingeschränkt ist. Die Sprödigkeit dieser Materialen führt weiterhin oft zu hohen Ausstoßungskräften an den Tablettierwerkzeugen, verbunden mit einem erhöhtem Verschleiß der Preßwerkzeuge, aber auch zur verstärkten Maschinenbelastung mit den damit verbundenen Ausfallzeiten und Ersatzbeschaffungen.

US 2009/087485 Al befasst sich mit direkt verpressbaren Zubereitungen mit guter mechanischer Härte und geringer Zerfallszeit. Die Zubereitungen bestehen aus sprühgetrocknetem Calcium Silikat, Mannitol und Sorbitol, die zu Tabletten verpresst werden und eine geeignete Härte, Friabilität und Zerfallszeit aufweisen.

Aufgabe der vorliegenden Erfindung ist es also, ein Verfahren zur Verfügung zu stellen, wodurch auch problematische Wirk- und Hilfsstoffe in einem Verfahren durch Direktverpressen zu Tabletten verarbeitet werden können. Eine weitere Aufgabe der vorliegenden Erfindung besteht darin, aus den entsprechenden Wirk- und Hilfsstoffen frei fließende gut komprimierbare Massen herzustellen, die sich in einfacher Weise zu Tabletten verpressen lassen.

Die Lösung der vorliegenden Aufgabe erfolgt an sich dadurch, dass man durch die geschickte Kombination und/oder physikalische Modifikation der Hauptbestandteile einer Tablettenrezeptur frei fließende, gut komprimierbare Massen herstellt, die auch unter Zusatz der weniger gut tablettierbaren Rezepturkomponenten eine direkte Verpressung erlauben. Insbesondere werden die Eigenschaften der verschiedenen zugesetzten Komponenten so ausgenutzt, dass diese DC-Materialen einfach zu verarbeiten sind, physiologisch und chemisch inert sind und sich bereits bei möglichst niedrigen Presskräften zu Tabletten mit sehr guten Tablettenhärten bei gleichzeitig ausreichend schnellen Zerfallszeiten verarbeiten lassen.

Gegenstand der vorliegenden Erfindung ist demzufolge eine direkt verpressbare Zusammensetzung für die Tablettenherstellung, die dadurch gekennzeichnet ist, dass sie aus Calciumhydrogenphosphat Dihydrat und einem plastischen Tablettierhilfsmittel gemäß Anspruch 1 besteht.

Diese direkt verpressbare Zusammensetzung für die Tablettenherstellung, besteht insbesondere aus Calciumhydrogenphosphat Dihydrat und mindestens einem Polyol.

Besonders bevorzugt besteht diese Zusammensetzung aus Calciumhydrogenphosphat Dihydrat und mindestens einem Polyol, ausgewählt aus der Gruppe Mannit, Sorbit, Xylit und Erythrit. Insbesondere bevorzugt sind in dieser Zusammensetzung Calciumhydrogenphosphat Dihydrat und die Polyole Mannit und Sorbit enthalten.

Gute Eigenschaften werden für direkt verpressbare Zusammensetzungen gefunden, wenn zur Herstellung der Tabletten, Calciumhydrogenphosphat Dihydrat, Mannit und Sorbit in einem Gewichtsverhältnis im Bereich von 50 : 40 : 10 und 85 : 10 : 5 vorab co-sprühgranuliert werden.

Besonders gute Eigenschaften haben sich gezeigt, wenn zur Herstellung dieser direkt verpressbare Zusammensetzung für die Tablettenherstellung, Calciumhydrogenphosphat Dihydrat, Mannit und Sorbit in einem Gewichtsverhältnis im Bereich von 60 : 30 : 10 bis 70 : 20 : 10 miteinander kombiniert werden.

Direkt verpressbare Zusammensetzungen, wie hier beschrieben, zeichnen sich durch einen Fließwinkel im Bereich von 29 bis 42° aus, insbesondere von 29 bis 35°, besonders bevorzugt im Bereich von 29 bis 30°, wodurch sie besonders geeignet sind zur Einzeldosierung in die Matrizen der Tablettiermaschinen bei der Tablettierung oder zur maschinellen Sacchetabfüllung.

Erfindungsgemäße direkt verpressbare Zusammensetzungen weisen eine Schüttdichte im Bereich von 0,55 - 0,75 g/ml auf bei einer Stampfdichte im Bereich von 0,73 - 0,90 g/ml. Diese Eigenschaften der pulverförmigen, direkt verpressbaren Zusammensetzung sind verbunden mit einer Kornverteilung von 8 bis 47 Gew.-% im Bereich von 50 bis 100 µm, 30 bis 68 Gew.-% im Bereich 100 bis 200 µm, 6 bis 44 Gew.-% im Bereich von 200 bis 315 µm und 0,6 bis 12 Gew.-% im Bereich von 315 - 500µm, wobei der Gewichtsanteil der Kornfraktion <50 µm nicht mehr als 7 Gew.-% und der Gewichtsanteil der Kornfraktion >500µm nicht mehr als 4 Gew.-% beträgt. Vorteilhafter Weise besitzt die Zusammensetzung einen hohen Calciumgehalt im Bereich von 11 bis 20 Gew.-%. Wird die erfindungsgemäße Zusammensetzung mit einer Preßkraft von 20 kN komprimiert, werden Tabletten mit Härten von > 250 N erhalten, verbunden mit einer Ausstoßkraft <210 N, einer Friabilität <0,12 %, einer Zerfallszeit <735 Sekunden. Insbesondere werden nach Kompression mit einer Preßkraft von 20 kN Preßlinge mit Härten von >300N erhalten, verbunden mit einer Ausstoßkraft von <110 N, einer Friabilität von <0,06 % und einer Zerfallszeit von <620 Sekunden. Wird die erfindungsgemäße Zusammensetzung mit einer Preßkraft von 30 kN komprimiert, werden Preßlinge mit Härten von >430N erhalten, verbunden mit einer Ausstoßkraft von <130N, einer Friabilität von maximal 0,08% und einer Zerfallszeit von <480 Sekunden. Dementsprechend ist auch eine Zusammensetzung bzw. Formulierung Gegenstand der vorliegenden Erfindung, welche die so charakterisierte direkt verpressbare Zusammensetzung enthält und als feste Form oder Komprimat vorliegt. Solche eine Zusammensetzung bzw. Formulierung kann einen oder mehrere nicht wasserlösliche und/oder wasserlösliche Zusätze homogen verteilt enthalten. Diese Zusätze sind bevorzugt ausgewählt aus der Gruppe pharmazeutische Wirkstoffe, Pflanzenextrakte, Süßstoffe, Farbstoffe, Zitronensäure, Vitamine und Spurenelemente. Weiterhin kann eine solche erfindungsgemäße Zusammensetzung bzw. Formulierung einen oder mehrere pharmazeutische Wirkstoffe aus der Gruppe der Analgetica enthalten, insbesondere aber auch einen oder mehrere Süßstoffe, ausgewählt aus der Gruppe Acesulfam K, Aspartam^{®}, Saccharin, Cyclamat, Sucralose und Neohesperidin DC enthält.

Gegenstand der vorliegenden Erfindung ist auch ein Verfahren zur Herstellung dieser direkt verpressbaren Zusammensetzungen für die Tablettenherstellung, indem eine Lösung oder Suspension, enthaltend Calciumhydrogenphosphat Dihydrat, Mannit und Sorbit in einem Gewichtsverhältnis im Bereich von 50 : 40 : 10 und 85 : 10 : 5, bevorzugt in einem Gewichtsverhältnis im Bereich von 60 : 30 : 10 und 70 : 20 : 10 , in Wasser, wobei in 4 Teilen Wasser 4 Teile Feststoff gelöst, bzw. suspendiert werden, entweder chargenweise (batch) oder kontinuierlich in einem Wirbelschichtgranulator einem Co-Sprühgranulationsprozess unterworfen werden.

Durch Versuche wurde gefunden, dass die Kombination des spröden Calciumhydrogenphosphat Dihydrats mit einem im Vergleich dazu eher plastischen Material, wie z.B einem Polyol, zu einer deutlich verbesserten Tablettenqualität führt, was sich einerseits in einer erheblich verbesserten Kompressibilität zeigt, andererseits aber gleichzeitig Tabletten mit schneller Tablettenzerfallszeit erhalten werden. Insbesondere wurde gefunden, dass aus einer Kombination, bestehend aus etwa 50 - 85 Gew.% pulverförmiges Calciumhydrogenphosphat Dihydrat , etwa 10-30 Gew.% Mannit und etwa 5 - 15% Gew.% Sorbit ein entsprechend verbessertes Produkt erhalten werden kann. Insbesondere wird durch einen Co-Sprühgranulationsprozess ein hinsichtlich Fließverhalten, Kompressibilität, Zerfallseigenschaften und anderer galenischer Kennzahlen optimales Produkt für den Direkttablettierungsprozess erhalten. Das erfindungsgemäße Material zeigt deutlich bessere Verarbeitseigenschaften als es z.B. durch einfache physikalische Mischungen selbst unter Verwendung von direkttablettierbaren Einzelkomponenten möglich wäre. Es wurde weiterhin gefunden, dass nur durch Zusatz einer gewissen Menge an Sorbit die galenischen Eigenschaften dieser Co-Sprühprodukte verbessert werden.

Das Verhältnis der drei oben genannten Bestandteile muß zudem in einem optimierten Bereich gehalten werden, um die verbesserten Preßkraft/Härte- bzw. Härte/Zerfallszeit-Profile zu erhalten. Insbesondere wurde gefunden, dass die verbesserten Eigenschaften erhalten werden, wenn das Gewichtsverhältnis in einem Bereich zwischen etwa 60: 30 : 10 und 70: 20 : 10 bezogen auf das Verhältnis von Calciumhydrogenphosphat Dihydrat zu Mannit zu Sorbit liegt. In diesem Bereich liefern die entsprechende Zusammensetzungen besonders verbesserte Preßkraft/Härte-bzw. Härte/Zerfallszeit-Profile. Es liegt in dieser Zusammensetzung offensichtlich ein ausgewogenes Verhältnis zwischen der Plastizität der Polyole und Sprödigkeit des Calciumhydrogenphosphat Dihydrats vor, welches die sehr guten Verpreßeigenschaften bedingt.

Mit dem erhaltenen Material erhält der Galeniker ein hinsichtlich der Direkttablettierungseigenschaften optimiertes Produkt mit dessen Hilfe auch per se schlecht tablettierbare Wirkstoffe diesem einfachen Tablettierverfahren zugänglich werden. Durch seinen hohen Calcium- und Phosphatgehalt ist das Produkt außerdem zur Formulierung von mit Calcium und Phosphor angereicherten Preßlingen z.B. in Kautabletten zur Nahrungsergänzung von Interesse. Diese Verwendung bietet sich insbesondere auch deshalb an, da das Material durch die Feinstverteilung des feinkörnigen, von Natur aus unangenehm sandig schmeckenden und praktisch unlöslichen Calciumhydrogenphophat Dihydrats in einer Matrix aus den süß und kühl schmeckenden Polyolen sehr gute sensorische Eigenschaften erhält.

Durch die vorliegende Erfindung wird also eine direktverpreßbare Tablettiermasse, bzw. ein Tablettierhilfsmittel auf Basis von Calciumhydrogenphosphat Dihydrat mit verbesserten Eigenschaften zur Verfügung gestellt, das durch eine Co-Sprühgranulation von Calciumhydrogenphosphat Dihydrat, Mannit und Sorbit erhalten wird.

Entsprechende erfindungsgemäß zusammengesetzte Hilfsmittel zur Herstellung von Tabletten, bestehend aus Calciumhydrogenphosphat Dihydrat/Mannit/ Sorbit, die durch Co-Sprühgranulation erhalten werden, sind einsetzbar zur Herstellung von direktverpreßbaren Tablettiermassen.

Wie aus den sensorischen Eigenschaften von Calciumhydrogenphosphat Dihydrat zu schließen ist, wird eine sehr homogenen Verteilung des in Wasser bei neutralem pH-Wert kaum löslichen Calciumhydrogenphosphat-Dihydrats in einer Matrix aus den beiden in Wasser löslichen Polyolen Mannit und Sorbit erhalten. Diese homogene Verteilung kann durch ein Co-Sprühgranulationsverfahren aller Komponenten aus wässriger Lösung bzw. Suspension in einem Wirbelbett erzielt werden.

Vorteilhafter Weise können dadurch neben der angenehmen Sensorik (Mundgefühl) auch die Direktverpressungseigenschaften verbessert werden.

In diesem Zusammenhang zeigen die erfindungsgemäßen cosprühgranulierten Zusammensetzungen, bestehend aus Calciumhydrogenphosphat Dihydrat, Mannit und Sorbit, eine Reihe von unerwarteten Vorteilen:
1. Verbesserte Eigenschaften während des Verpressens zu Tabletten: Während Calciumhydrogenphosphat Dihydrat und Mannit üblicherweise recht schwierig zu Tabletten verpreßt werden können, weist die Dreierkombination Calciumhydrogenphosphat DihydratlMannit/Sorbit sehr gute Eigenschaften beim Direktverpressen auf, insbesondere wenn die Komponenten in bestimmten Mengenverhältnissen vorab miteinander einer Co-Sprühgranulation unterzogen werden. Die so erhaltenen Produkte lassen sich anschließend zu Tabletten mit verbesserten Eigenschaften verarbeiten.
   - Besonders gute Preßkraft-Härte-Relationen werden gefunden, wenn die drei Komponenten Calciumhydrogenphosphat Dihydrat, Mannit und Sorbit in einem Verhältnis im Bereich zwischen 50 : 40 : 10 und 85 : 15 : 5 , insbesondere im Bereich zwischen 60 : 30 : 10 und 70 : 20 : 10 miteinander co-sprühgranuliert werden. Diese Mischungsverhältnisse stellen offensichtlich ein optimales Verhältnis der spröden Eigenschaften des Calciumhydogenphosphat Dihydrats und der plastischen Eigenschaften einer Mannit/Sorbit-Kombination dar für die Komprimierung während des Verpressens. In Versuchen zeigten insbesondere entsprechende Produkte, in denen Calciumhydrogenphosphat Dihydrat, Mannit und Sorbit in einem Mischungsverhältnis von 60 : 30 : 10 oder von 70 : 20 : 10 miteinander co-gesprüht wurden besonders gute Eigenschaften. Zusammensetzungen, in denen ein höherer Anteil an Calciumhydrogenphosphat Dihydrat eingesetzt wird während der Co-Sprühung oder in denen zur Co-Sprühung der Anteil der Polyole Mannit oder Sorbit erhöht wird, weisen schlechtere Kompressibilitäten auf. Aber auch wenn in den Zusammensetzungen nur eines der beiden Polyole gemeinsam mit Calciumhydrogenphosphat Dihydrat co-gesprüht wird, führt dieses zu einer Abnahme der Kompressibilität.
   - Gegenüber handelsüblichem, direktverpreßbarem (DC) Calciumhydrogenphosphat Dihydrat sind die Eigenschaften der Dreierkombinationen, insbesondere der beiden bevorzugten Dreierkombinationen im Mischungsverhältnis von 60 : 30 : 10 oder von 70 : 20 : 10, während des Direktverpressens erheblich verbessert. Aber auch im Vergleich zu einer physikalischen Mischung, bestehend aus handelsüblichem DC-Calciumhydrogenphosphat Dihydrat und den per se sehr gut direkt verpreßbaren DC-Mannit-Typen (Parteck M 200) und DC-Sorbit (Parteck SI 150) im Verhältnis 70 : 20 : 10, zeigen sich stark verbesserte Eigenschaften.
2. Kurze (schnelle) Zerfallszeiten der Preßlinge auch in hohen Härtebereichen
   Durch die Co-Sprühung als Dreierkombination mit mindestens 50 und höchstens 85 Gew.-% Calciumhydrogenphosphat Dihydrat werden bei hohen Tablettenhärten ohne Zusatz von zerfallsfördenden Komponenten (Sprengmittel) gleichzeitig kürzere Zerfallszeiten erzielt im Vergleich zu Zusammensetzungen mit gleichen Tablettenhärten, die mittels Verpressen von mechanischen Mischungen bei höherem Preßdruck erhalten worden sind. Wird jedoch in der co-sprühgranulierten Zusammensetzung der Calciumhydrogenphosphat-gehalt noch darüber hinaus erhöht, führt dieser weitere Dihydrat-Zusatz zu einer Zerfallsverzögerung.
   - Die beiden bevorzugten Zusammensetzungen mit einem Calciumhydrogenphosphat Dihydrat : Mannit : Sorbit-Verhältnis von 60 : 30 : 10 oder 70 : 20 : 10 zeigen über einen deutlich weiteren Tablettenhärtebereich kurze Zerfallszeiten, und zwar vergleichsweise deutlich kürzere Zerfallszeiten als von im Handel erhältlichen, zu Tabletten verpressten DC-Calciumhydrogenphosphat Dihydraten oder von entsprechenden, verpressten, physikalischen Mischungen mit DC-Mannit-Typen (Parteck M 200) und DC-Sorbit (Parteck SI150).
3. Mechanische Stabilität der resultierenden Tabletten
   Die mechanische Stabilität pharmazeutischen Formulierungen in Form von Granulaten oder Tabletten wird u. a. anhand ihrer Friabilität beurteilt. Friabilität ist das Maß in Gewichtsprozent für den mechanischen Abrieb der Tabletten unter mechanischer Beanspruchung. Von der Herstellung bis zum endgültigen Verbrauch sind Tabletten physischen Belastungen ausgesetzt. Sie müssen daher so entwickelt werden, dass sie die erhaltenen Stöße möglichst unbeschadet überstehen.
   Um festzustellen, wie die zu prüfenden Tabletten diesen Belastungen widerstehen und um Rückschlüsse auf die Weiterverarbeitung (z.B. Lackierung, Dragierung, Konfektionierung) ziehen zu können, werden daher Friabilitäts- oder Abriebtest durchgeführt. Dabei werden die Tabletten nach dem Prinzip einer sich wiederholenden Bewegung in Roche-Friabilator- oder Abriebtrommeln geprüft. Die Testbedingungen, wie Anzahl der Muster, Anzahl der Umdrehungen und - Prüfgeschwindigkeit sind in den Pharmakopöen definiert. Als Abrieb wird die Masse bezeichnet, welche die Tabletten durch die mechanische Belastung verlieren.
   Zur Bestimmung der Friabilität sind im Handel verschiedene Geräte in unterschiedlichen Ausführungsformen erhältlich. Das ERWEKA-Prüfgerät TDR 100 ist ein halbautomatisches Kombinationssystem aus einem ERWEKA Abrieb-/Friabilitätstestgerät und einer Sartorius Analysenwaage. Die Steuerung des Systems erfolgt vom ERWEKA Abrieb-/Friabilitätstestgerät (Erweka Apparatebau, Heusenstamm).
   Andere Geräte sind :
   Friabilitätstester Typ TAP Nr. 43651, Erweka Apparatebau, Heusenstamm
   Abriebtester vom Arzneimittelwerk Dresden, Dresden,
   Friabilator Typ PTF 1, Fa. Pharmatest,
   Roche Friabilator, J. Engelsmann AG, Ludwigshafen/Rhein

   Die Friabilität der zu testenden Tabletten wird in diesen Geräten nach Verfahren geprüft, wie in Ph. Eur. Nachtrag 2001 oder Ph. Eur. 6. Ausgabe Grundwerk 2008 unter "2.9.7 Friabilität von nicht überzogenen Tabletten" beschrieben ist.
   Für die Beurteilung kann eine festgelegte Anzahl staubfreier Tabletten in eine Trommel mit einer Schikane für eine bestimmte Zeit und bei festgelegter Drehzahl bewegt werden. Anschließend wird der Masseverlust der von Staub befreiten Tabletten in Prozent bestimmt.
   In den Versuchen der vorliegenden Erfindung wurde die Friabilität der durch Verpressen hergestellten Tabletten bestimmt, indem wie in der Ph. Eur. 6. Ausgabe Grundwerk 2008 unter 2.9.7 beschrieben, Tabletten in einem Friabilator nach Roche auf ihren Abrieb untersucht wurden. Es wurden mit dem Gerät jeweils 100 Umdrehungen durchgeführt, wobei die Umdrehungsgeschwindigkeit bei 25 +/-1 min⁻¹ lag.
   Durch die Messungen wurde gefunden, dass die Friabilitäten von co-gesprühten Zusammensetzungen mit einem Calciumhydrogenphosphat Dihydrat : Mannit : Sorbit-Verhältnis von 70 : 20 :10, die bei unterschiedlichen Preßkräften zu Tabletten konfektioniert worden sind, wesentlich geringer sind als bei Tabletten, in denen allein DC-Calciumhydrogenphosphat Dihydrat eingesetzt worden ist. Auch wurde bei diesen, erfindungsgemäßen Tabletten kein "Deckeln" beobachtet. Für co-sprühgranulierte Zusammensetzungen mit einem Gewichtsverhältnis von Calciumhydrogenphosphat Dihydrat : Mannit : Sorbit im Bereich zwischen 50 : 40 : 10 und 85 : 10 : 5 , insbesondere im Bereich zwischen 60 : 30 : 10 und 70 : 20 : 10 wurden vergleichbar verbesserte Ergebnisse beobachtet. Besonders gute Ergebnisse wurden für Zusammensetzungen gefunden, in denen das Verhältnis im Bereich zwischen zwischen 60 : 30 : 10 und 70 : 20 : 10 liegt. Aufgrund dieser verbesserten Eigenschaften liegen Zusammensetzungen vor, die eine sehr sichere Handhabung der Preßlinge, bzw. Tabletten im weiteren Verarbeitungsprozess z.B. in Verpackungsmaschinen oder Coatinganlagen ermöglichen und bei der Blisterentnahme durch den Patienten sicher gehandhabt werden können.
4. Geringe Ausstoßkräfte d.h. Schonung der Stempelwerkzeuge und Tablettiermaschinen

Die erfindungsgemäßen Zusammensetzungen weisen nicht nur eine verringerte Friabilität auf. Auch während der Tablettierung machen sich die verbesserten Eigenschaften bemerkbar. Insbesondere sind bei der Tablettierung von erfindungsgemäßen Calciumhydrogenphosphat Dihydrat /Mannit /Sorbit-Zusammensetzungen bei allen geprüften Preßkräften die erforderlichen Ausstoßkräfte minimiert. Minimierte Ausstoßkräfte werden insbesondere für Calciumhydrogenphosphat Dihydrat /Mannit /Sorbit-Zusammensetzungen im Verhältnis 70 : 20 : 10 gefunden. Dieses bedeutet, dass bei der Tablettierung einer solchen Dreierkombinationszusammensetzung eine optimale Schonung der Preßwerkzeuge und Maschinen bei diesem Vorgang erzielt wird.

Bei Verwendung erfindungsgemäßer Zusammensetzungen während der Tablettierung klebten diese weder am Stempel noch an den Matrizen der Tablettiermaschine noch zwischen dem Stempel und den Matrizen. Die erfindungsgemäßen Zusammensetzungen neigen auch nicht dazu, sich an den Stempeln und Matrizen anzulagern und dadurch Reibung zwischen dem Stempel und der Matrize zu verursachen. Daher lassen sie sich mit verringertem Druck aus den Tablettiermatrizen ausstoßen.

Die erfindungsgemäßen Zusammensetzungen lassen sich fortlaufend und stabil für eine lange Zeit auf entsprechenden Tablettiermaschinen industriell verarbeiten, ohne dass es zu einem sogenannten Rauhlaufen der Maschinen kommt.

Bereits durch die Herstellung der Vorprodukte für die Tablettenherstellung durch Co-Sprühgranulation von Calciumhydrogenphosphat Dihydrat, Mannit und Sorbit in erfindungsgemäßen Gewichtsverhältnissen werden Zusammensetzungen mit einem hohen Calciumhydrogenphosphat-Anteil erhalten, die auch zur Calcium- und Phosphoranreichung in Lebensmitteln, als Nahrungsergänzungsmittel oder in pharmazeutischen Zubereitungen, insbesondere in Form von Pulversacchets, Preßlingen oder in Kapseln verwendet werden können. Weiterhin ist der Fließwinkel im Bereich von 29 bis 42°, insbesondere von 29 bis 35°, besonders bevorzugt im Bereich von 29 bis 30°, des hergestellten Materials für die Weiterverarbeitung optimal. Das Material ist damit beispielsweise hervorragend zur Einzeldosierung in die Matrizen der Tablettiermaschinen bei der Tablettierung oder zur maschinellen Sacchetabfüllung geeignet.

Zur Durchführung der Co-Sprühgranulation werden vorab wässrige Lösungen bzw. Suspensionen der verschiedenen Komponenten hergestellt. Bevorzugt wird mit 50-%igen [50%ig(m/m)] Lösungen bzw. Suspensionen gearbeitet, wobei die Prozentangaben sich auf die jeweiligen Gewichtsprozente beziehen. Dabei werden die Trockensubstanzen in vollentsalztem Wasser gelöst, bzw. suspendiert. Zur Durchführung der Co-Sprühganulation werden die mengenmäßigen Verhältnisse des Calciumhydrogenphosphat Dihydrats zu den Polyolen Mannit und Sorbit in Lösung so eingestellt, dass sich die gewünschten gewichtsmäßigen Verhältnisse in der co-gesprühten Substanz im erfindungsgemäßen Verhältnis zueinander befinden. Zur Herstellung der Sprühlösung wird in einem Ansatzgefäß die vorausberechnete Menge vollentsalztes Wasser vorgelegt. In dem Wasser werden unter Rühren bei 20 - 25°C die Polyole Sorbit und Mannit bis zur vollständigen Auflösung eingerührt. In diese klare Lösung wird ebenfalls unter Rühren das Calciumhydrogenphosphat Dihydrat eingetragen und die weiße Suspension solange gerührt bis alle eventuell gebildeten Agglomerate zerfallen sind. Diese Lösung/Suspension wird unter ständigem Rühren in der Co-Sprühgranulation versprüht.

Um ein möglichst homogen verteiltes Calciumhydrogenphosphat Dihydrat in der Polyolmatrix zu erhalten, kann zunächst in einem Batch-Verfahren ein Startgranulat erzeugt (Vorsprühung) werden, wovon dann jeweils eine kleine Menge zur Vorlage in der Wirbelschicht für einen oder weitere Co-Sprühgranulationsprozesse (Hauptsprühungen) eingesetzt werden kann. Auf diese Weise kann der Anteil an nicht homogen verteiltem Calciumhydrogenphosphat in der Polyolmatrix immer weiter bis zu einem vernachlässigbaren Anteil reduziert werden.

Wesentlich einfacher ist es natürlich, wenn als Startgranulat co-gesprühtes Material der gewünschten Zusammensetzung eingesetzt wird, welches aus vorhergehenden Sprühungen entnommen worden ist und in der Wirbelschicht vorgelegt werden kann. Die Aufsprühung erfolgt dann wie in den folgenden Beispielen für die Hauptsprühung beschrieben.

Die kontinuierliche Herstellung wird in ähnlicher Weise durchgeführt wie in den Schriften EP 1 453 781A1, EP 1 319 644 A1 und WO 00/76650 A1 beispielsweise für die Herstellung von alpha- oder beta-Mannit beschrieben. Insbesondere erfolgt die kontinuierliche Herstellung der erfindungsgemäßen Zusammensetzungen in einem Wirbeischichtgranulator mit Pulverrückführung und kontinuierlicher Entnahme von Produkt, wobei die mittlere Korngröße des entstandenen Produkts durch den Luftstrom im Wirbelbett gesteuert wird.

Durch diese Cosprühgranulation wird eine sehr homogene Verteilung des in Wasser bei neutralem pH-Wert kaum löslichen Calciumhydrogenphosphat Dihydrats in einer Matrix aus den beiden in Wasser löslichen Polyolen Mannit und Sorbit erzielt. Diese homogene Verteilung wird durch ein Co-Sprühgranulationsverfahren aller Komponenten aus wässriger Lösung bzw. Suspension in einem Wirbelbett erreicht. Neben einem Produkt mit einer angenehmen Sensorik (Mundgefühl) wird auf diese Weise auch ein Produkt mit sehr guten Direktverpressungseigenschaften erhalten.

Das Prinzip der Co-Sprühgranulationsverfahrens und die Konstruktion der Anlage ergeben sich aus den Patentschriften EP 1 453 781 (beta-Mannit), EP 1 319 644 (alpha-Mannit) und WO 00/76650.

Durch Variation der Verfahrensparameter Sprühdruck, Sprühmenge, zurückgeführte Pulvermenge, Heißluftstrom und Temperatur der Heißluft lassen sich die gewünschten Partikelgrößen herstellen. Gegebenenfalls. kann auch durch eine Siebklassierung am Austrag der Anlage eine Korneingrenzung vorgenommen werden. Grobkorn kann über einen Mahlventilator zerkleinert zurück in das Sprühsystem geführt werden.

Prinzipiell erfolgt die Herstellung der erfindungsgemäßen Zusammensetzungen in ähnlicher Weise wie sie in den Schriften EP 1 453 781A1, EP 1 319 644 A1 für die Herstellung von alpha- und beta-Mannit oder in WO 00/76650 A1 beschrieben ist. Und zwar erfolgt die Co-Sprühgranultion in einem Wrbelschichtgranulator mit Pulverrückführung, worin das Versprühen der Lösungen, bzw. Suspensionen mittels Zweistoffdüsen, über die gleichzeitig rückgeführtes Pulver in die Sprühzone transportiert wird.

Zu diesem Zweck ist der Sprühdruck der Zweistoffdüsen im Bereich von 2 - 4 bar einzustellen; vorzugsweise wird im Bereich 2,5 - 3,5 bar gearbeitet. Die der Zweistoffdüse zugeführte Menge Heißgas ist so zu regulieren, dass bis zu etwa 1,5 - 3m³/(h kg Suspension) mit einer Temperatur von etwa 80 - 110°C gefördert werden.

Die Pulverrückführung ist so einzustellen dass eine Feststoffrückführung im Bereich 0,2 - 2,0 kg Feststoff/(h kg Suspension) erfolgt. Vorzugsweise wird im Bereich von 0,5 - 1,5 kg Feststoff /(h kg Lösung) gearbeitet. Besonders günstig gestaltet sich der Prozess, wenn die Feststoffrückführung im Bereich von 0,5 - 1,0 kg/(h kg Lösung) liegt.

Zur Durchführung des Verfahrens muss in die Anlage vorgewärmte Luft eingespeist werden. Gute Ergebnisse werden erzielt, wenn die der Anlage zugeführte Luft auf eine Temperatur im Bereich von 45 - 120 °C vorgewärmt wird. Günstig ist es für den erfindungsgemäßen Prozess, wenn die Zuluft eine Temperatur im Bereich von 65 - 110 °C aufweist. Besonders vorteilhaft für die Bildung eines co-gesprühgranulierten Calciumhydrogenphosphat/Mannit/Sorbits mit guten Tablettierungseigenschaften ist es, wenn die Temperatur der eingespeisten Zuluft im Bereich von 70 - 100 °C liegt. Die zugeführte Zuluftmenge ist erfindungsgemäß so zu regeln, dass 1000 - 2000 m³/m² pro Stunde, insbesondere 1200 - 1700 m³/m² pro Stunde, in die Anlage eingespeist werden.

In Verbindung mit den übrigen eingestellten Parametern liegen günstige Verfahrensbedingungen vor, wenn der Luftstrom in der Anlage so geführt wird, dass sich die Ablufttemperatur im Bereich von 30 - 50 °C einstellt und sich die Temperatur des gebildeten Produkts auf eine Temperatur in demselben Bereich bis zu 50 °C einstellt.

Weiterhin hat es sich als günstig herausgestellt, die Verfahrensbedingungen so zu regulieren, dass sich die im Wirbel- oder Fließbett befindliche Pulvermenge auf eine Bettmenge von 50 - 150 kg/m² Bett einstellt. Besonders günstig ist es, wenn die Bettmenge im Bereich von 80 - 120 kg/m² Bett liegt.

Durch eine gezielte Pulverrückführung sowohl durch Pulverentnahme aus dem Fließbett als auch durch Rückführung einer sehr feinen Pulverfraktion, welche bei der Konfektionierung d.h. Homogenisierung der Partikelgröße durch Siebung bei Abfüllung des hergestellten Produktes anfällt, lässt sich der Prozeß bezüglich der gewünschten Partikelverteilung steuern.

Es ist auch möglich, vor der Rückführung vorab Pulver mit größeren Partikelquerschnitten im Mahlventilator der Sprühgranulationseinheit zu zerkleinern, sofern in einem ein Wirbelschichtgranulator gearbeitet wird, wie in EP 1 453 781A1 oder EP 1 319 644 A1 beschrieben ist.

Durch die besondere Herstellungsweise in einem Co-Sprühgranulationsverfahren werden direkt verpressbare Zusammensetzungen mit einer Schüttdichte im Bereich von 0,55 - 0,75 g/ml und einer Stampfdichte im Bereich von 0,73 - 0,90 g/ml erhalten. Diese Eigenschaften sind verbunden mit einer Kornverteilung von 8 bis 47 Gew.-% im Bereich von 50 bis 100 µm, 30 bis 68 Gew.-% im Bereich 100 bis 200 µm, 6 bis 44 Gew.-% im Bereich von 200 bis 315 µm und 0,6 bis 12 Gew.-% im Bereich von 315 - 500µm, wobei der Gewichtsanteil der Kornfraktion <50 µm nicht mehr als 7 Gew.-% und der Gewichtsanteil der Kornfraktion >500µm nicht mehr als 4 Gew.-% beträgt. Je nach dem Gewichtsanteil von mit versprühten Polyol während der Co-Sprühgranulation besitzt die Zusammensetzung einen Calciumgehalt im Bereich von 11 bis 20 Gew.-%.

Durch Untersuchungen der Tablettierungseigenschaften der erfindungsgemäßen direkt verpressbaren Zusammensetzungen wurde gefunden, dass die erfindungsgemäße Zusammensetzung mit einer Preßkraft von 20 kN zu Tabletten mit Härten von > 250 N komprimiert werden kann, verbunden mit einer Ausstoßkraft <210 N, einer Friabilität <0,12 %, einer Zerfallszeit von <735 Sekunden. Insbesondere werden nach Kompression mit einer Preßkraft von 20 kN Preßlinge mit Härten von >300N erhalten, verbunden mit einer Ausstoßkraft von <110 N, einer Friabilität von <0,06 % und einer Zerfallszeit von <620 Sekunden. Wird die erfindungsgemäße Zusammensetzung dagegen mit einer Preßkraft von 30 kN komprimiert, weisen die Preßlinge Härten von >430N auf, verbunden mit einer Ausstoßkraft von <130N, einer Friabilität von maximal 0,08% und einer Zerfallszeit von <480 Sekunden.

Erfindungsgemäß kann die direkt verpressbare Zusammensetzung gemäß der vorliegenden Erfindung in eine Zusammensetzung bzw. Formulierung eingebracht werden, welche als feste Form oder Komprimat vorliegt. Als pharmazeutisch einsetzbare Zusammensetzung bzw. Formulierung kann diese wiederum einen oder mehrere nicht wasserlösliche und/oder wasserlösliche Zusätze homogen verteilt enthalten. Die homogene Verteilung kann entweder durch vorheriges intensives Vermischen mit der direkt verpressbaren Zusammensetzung erzeugt werden, bevor die Tablettierung oder Konfektionierung erfolgt. Die homogene Verteilung kann aber auch erzielt werden durch gemeinsames Co-Sprühgranulieren unter geeigneten Bedingungen. Die wasserlöslichen oder nicht wasserlöslichen Zusätze werden insbesondere ausgewählt aus der Gruppe pharmazeutische Wirkstoffe, Pflanzenextrakte, Süßstoffe, Farbstoffe, Zitronensäure, Vitamine und Spurenelemente. Diese Zusätze werden so ausgewählt, dass sie in der Kombination der Einzelkomponenten der Zusammensetzung stabil und lagerfähig sind. Insbesondere kann eine solche erfindungsgemäße Zusammensetzung bzw. Formulierung einen oder mehrere pharmazeutische Wirkstoffe aus der Gruppe der Analgetica enthalten, weiterhin aber auch einen oder mehrere Süßstoffe, ausgewählt aus der Gruppe Acesulfam K, Aspartam^{®}, Saccharin, Cyclamat, Sucralose und Neohesperidin DC, um den Geschmack zu verbessern.

Zum besseren Verständnis und zur Verdeutlichung der Erfindung werden im folgenden Beispiele gegeben, die im Rahmen des Schutzbereichs der vorliegenden Erfindung liegen. Diese Beispiele dienen auch zur Veranschaulichung möglicher Varianten. Aufgrund der allgemeinen Gültigkeit des beschriebenen Erfindungsprinzips sind diese Beispiele jedoch nicht geeignet, den Schutzbereich der vorliegenden Anmeldung nur auf diese zu reduzieren.

Zum besseren Verständnis sind, sofern Unklarheiten bestehen, auch die in der Beschreibung zitierten Literaturstellen und Patentschriften heranzuziehen, die hiermit als Teil der Offenbarung der vorliegenden Erfindung gelten sollen.

Die in den folgenden Beispielen und der Beschreibung sowie in den Ansprüchen gegebenen Temperaturen gelten immer in °C. Wo nicht anders bezeichnet sind Gehaltsangaben als Gew.-% bzw. Gewichtsverhältnisse aufgeführt.

Weiterhin versteht es sich für den Fachmann von selbst, dass sich sowohl in dem gegebenen Beispiel als auch in der übrigen Beschreibung die in den Zusammensetzungen enthaltenen Komponentenmengen in der Summe immer nur zu 100 Gew.-% bzw. mol-% bezogen auf die Gesamtzusammensetzung aufaddieren und nicht darüber hinausgehen können, auch wenn sich aus den angegebenen Prozentbereichen höhere Werte ergeben könnten. Sofern nichts anderes angegeben ist, gelten %-Angaben als Gew.-%, mit Ausnahme von Verhältnissen, die in Volumenangaben wiedergegeben sind.

### Beispiele

Zur Durchführung der erfindungsgemäßen Co-Sprühgranulation werden folgende Geräte und Verfahren zur Charakterisierung der Stoffeigenschaften eingesetzt:
1. Schüttdichte: gemäß DIN EN ISO 60: 1999 (Deutsche Fassung) - Angabe in den Tabellen als "g/ml"
2. Stampfdichte: gemäß DIN EN ISO 787-11: 1995 (Deutsche Fassung) - Angabe in den Tabellen als "g/ml"
3. Schüttwinkel: gemäß DIN ISO 4324: 1983 (Deutsche Fassung) - Angabe in den Tabellen als "Grad"
4. Hausner Faktor: Berechnung gemäß Ph.Eur. 6. Ausgabe, Grundwerk 2008, Absatz 2.9.36 "Kompressibilitätsindex und Hausner-Faktor"
5. Kompressibilitätsindex : Berechnung gemäß Ph.Eur. 6.Ausgabe Grundwerk 2008, 2.9.36, "Kompressibilitätsindex und Hausner-Faktor"; - Angabe in den Tabellen als "%"
6. Tablettierungsprüfung: 492,5g des auf seine Tablettierungseigenschaften zu prüfende Material werden mit 7,5g Parteck LUB MST (Magnesiumstearat pflanzlich) EMPROVE exp PhEur, BP, JP, NF, FCC Art.No. 1.00663 (Merck KGaA, Deutschland) versetzt; das Magnesiumstearat wird zuvor über ein 250µm Sieb abgelegt und 5 Minuten in einem verschlossenen Edelstahlbehälter (Fassungsvolumen: ca. 2 I, Höhe: ca. 19,5 cm, Durchmesser: ca. 12 cm; Außenmaße) auf einem Labor-Taumelmischer (Turbula, Fa. Willy A. Bachofen, Schweiz) gemischt. Die Verpressung zu 500 mg Tabletten (11 mm Stempel, rund, flach, mit Facette) erfolgt auf einer instrumentierten Excenter-Tablettiermaschine Korsch EK 0-DMS (Fa. Korsch, Deutschland) mit dem Auswertesystem Catman 5.0, Fa. Hottinger Baldwin Messtechnik - HBM (Deutschland).
   Je Preßkraft (Soll-Einstellungen:5+/-1, 10+/-1, 20+/-2 und 30+/-2 kN; die effektiv gemessenen Ist-Werte sind in den Beispielen angegeben) werden mindestens 100 Tabletten zur Auswertung der Preßdaten und der galenische Kennzahlen hergestellt.
7. Bestimmung der Tablettenhärte, Durchmesser und Höhen: Erweka TBH 30 MD; Fa. Erweka (Deutschland); Durchschnittsdaten aus jeweils 20 Tablettenmessungen pro Preßkraft
8. Tablettenabrieb: Friabilitätsprüfgerät Fa. Erweka (Deutschland); Geräteparameter und Ausführung der Messungen gemäß Ph.Eur. 6. Ausgabe, Grundwerk 2008, 2.9.7. "Friabilität von nicht überzogenen Tabletten"
9. Tablettenmasse: Durchschnittswert aus der Wägung von 20 Tabletten; Waage: Mettler AT 201, Fa. Mettler (Deutschland)
10. Tablettenzerfall: automatisierter Zerfallstester disi4 der Fa. Biomation (Deutschland); Medium: entsalztes Wasser bei 37°C; Geräteparameter und Ausführung gemäß Ph.Eur. 6.Ausgabe "Zerfallszeit von Tabletten und Kapseln" (mit Scheibe)
11. Bestimmung der Partikelgrößen als Trockensiebung über einen Siebturm Retsch AS 200 control ,g', Fa.Retsch (Deutschland); Substanzmenge: 40g +/- 2g; Siebzeit: 30 Minuten; Amplitude: 1mm; Intervall: 5 Sekunden; Durchmesser der verwendeten Siebeinsätze: 200 mm; Siebgrößen: 1000, 710, 500, 315, 200, 100, 50 und 32 µm; Angabe der Mengenverteilung pro Siebfraktion in den Tabellen als "Gew.% der Einwaage"
12. Calciumgehaltsbestimmung: Komplexometrische Titration mit Na-EDTA-Lösung und potentiometrischer Indikation oder Farbindikation. Die prinzipielle Vorgehensweise ist in der Fachliteratur beschrieben wie z.B. in G. Jander, K.F. Jahr, H. Knoll "Maßanalyse - Theorie und Praxis der klassischen und der elektrochemischen Titrierverfahren", Verlag Walter de Gruyter, 1973 ISBN 3 11 005934 7 oder in den Applikationsunterlagen der Titrations- und Indikatorelektrodenhersteller z.B. von der Firma Mettler-Toledo GmbH, Deutschland oder der Firma Metrohm, Schweiz.
   Vor der Titration werden die Proben (ca. 0,2 g Einwaage, genau gewogen) mit etwas vollentsalztem Wasser angeschlämmt und mit 5ml 25%iger Salzsäure in Lösung gebracht. 20,00 ml Titriplex(III)-Lösung 0,1 mol/L (Art.Nr. 1.08431; MERCK KGaA, Deutschland) werden zudosiert, mit vollentsalztem Wasser auf 70 ml aufgefüllt, eine Puffertablette (Art.Nr. 1.08430, MERCK KGaA, Deutschland) hinzugefügt und nach dem Auflösen der Puffertablette unter Rühren mit ca.10 ml Ammoniumpufferlösung pH 10 - 11 (Art.Nr. 1.09478, MERCK KGaA, Deutschland) ein pH-Wert von 10 - 11 eingestellt. Anschließend wird mit einer Zinksulfatlösung (0,1 mol/L) potentiometrisch rücktitriert - aus der verbrauchten Menge Titripelx(III). Lösung 0,1mol/L kann der Calciumgehalt stöchiometrisch berechnet werden.

### Rohstoffe zur Herstellung der erfindungsgemäßen Zusammensetzungen

Calciumhydrogenphosphat-Dihydrat feinst gepulvert geeignet für die Verwendung als Excipient
EMPROVE® exp Ph Eur, BP, USP, FCC, E 341 (Art.Nr. 1.02146, Merck KGaA, Deutschland),
Korngröße: 99% <63µm, gemessen durch Laserbeugung mit Naßdispergierung
Geräte/Methode: Malvern Mastersizer 2000, Naßmodul Hydro 2000 S, Direktzugabe,
Auswertemodel: Universal;
Medium: vollentsalztes Wasser,
Brechungsindex: Medium 1.33 (MIE Parameter), Fraunhofer;
Rührgeschwindigkeit: 2000 Upm,
Ultraschall: 100%,
Obscuration: 10-20%,
Messdauer: 7500 ms;
Ausführung gemäß technischem Handbuch und Spezifikationen des Herstellers

D(-)-Mannit geeignet für die Verwendung als Excipient EMPROVE® exp Ph Eur, BP, USP, JP, FCC, E 321 (Art.Nr. 1.05980, Merck KGaA, Deutschland)

Parteck® SI 400 (Sorbit) geeignet für die Verwendung als Excipient EMPROVE® exp Ph Eur, BP, NF, E 420 (Art.Nr. 1.03140, Merck KGaA, Deutschland)

### Vollentsalztes Wasser

### Vergleichssubstanzen:

Parteck^{®} M200 (Mannit) geeignet für die Verwendung als Excipient EMPROVE^{®} exp Ph Eur, BP, JP, USP, E 421 (Art.Nr. 1.00419, Merck KGaA, Deutschland)
Parteck^{®} SI 150 (Sorbit) geeignet für die Verwendung als Excipient EMPROVE^{®} exp Ph Eur, BP, JP, NF, E 420 (Art.Nr. 1.03583, Merck KGaA, Deutschland)
Emcompress^{®} Premium Dibasic Calcium Phosphate Dihydrate, USP, Calcium Hydrogen Phospahte Dihydrate, Ph. Eur. Dibasic Calcium Phospaphate, JP (JRS PHARMA GmbH & Co. KG, Deutschland) Batch No.: A71345A
DI-CAFOS Dicalcium phosphate 2-hydrate coarse powder, USP, FCC, Ph.Eurs., JP, E 341 (Product No.: C 92-14, Chemische Fabrik Budenheim KG, Deutschland) Material-No. 00000809 Batch-No.: B09094A
DI-TAB^{®} Dicalcium Phosphate Dihydrate, Unmilled USP, FCC, EP, E 341 (Innophos Inc., USA; bezogen über Univar GmbH, Essen, Deutschland) Lot 7016

### Allgemeine Durchführung der Co-Sprühgranulation

Eine Lösung bzw. Suspension aus 4 Teilen Wasser und 4 Teilen Feststoff, wobei der Feststoff aus 6 - 7 Teilen pulverförmigen Calciumhydrogenphosphat Dihydrat, 2 - 3 Teilen Mannit und 1 Teil Sorbit besteht, bzw. wobei der Feststoff das Verhältnis der pulverförmigen Ausgangsstoffe Calciumhydrogenphosphat Dihydrat, Mannit und Sorbit in dem gewünschten Verhältnis der herzustellenden Zusammensetzung aufweist, wird in einem Wirbelschichtgranulator einem Granulationsprozess (chargenweise oder kontinuierlich) unterworfen. Zur Verhinderung von Klebeeffekten im Granulator kann insbesondere auch bei Einsatz eines kontinuierlichen Prozesses eine teilweise Feststoffrückführung eingesetzt werden. Durch gegebenenfalls nachgeschaltete Siebverfahren kann ein Produkt mit einer definierten Kornverteilung bzw. Schütt- und Stampfdichte erhalten werden.

### Herstellung der Sprühlösungen bzw. Sprühsuspensionen:

Alle Sprühlösungen bzw. Suspensionen wurden als 50%ige Lösungen bzw. Suspensionen bezogen auf das Gewicht der Trockensubstanz und das Gewicht des vollentsalzten Wassers hergestellt. Die Verhältnisse des Calciumhydrogenphosphat Dihydrats zu den Polyolen ergeben sich aus den gewünschten Zusammensetzungen der gewünschten Endprodukte wie in Tabelle 1 angegeben.

In dem in einem Ansatzgefäß vorgelegten Wasser werden unter Rühren bei 20 - 25 °C die Polyole bis zur vollständigen Auflösung eingerührt. In diese klare Lösung wird ebenfalls unter Rühren das Calciumhydrogenphosphat Dihydrat eingetragen und die weiße Suspension solange gerührt bis alle eventuell gebildeten Agglomerate zerfallen sind. Diese Lösung/Suspension wird unter ständigem Rühren versprüht.

### Herstellung des Startmaterials für die Co-Sprühganulation:

Zum Anfahren der Anlage bedarf es einer primären Bettmenge um den Co-Sprühgranulationsprozess in Gang zu bringen. Dieses Startbett kann auf zwei Wegen erzeugt werden:
1. Man befüllt die Anlage zu Beginn des Sprühprozesses mit zurückgehaltenem aus vorhergehend erfindungsgemäß co-gesprühten Materialmengen
   oder
2. Man befüllt die Anlage mit einer physikalischen Mischung der gewünschten Komponenten in der herzustellenden qualitativen und quantitativen Zusammensetzung, d.h. mit pulverförmigen Calciumhydrogenphosphat Dihydrat; Mannit und Sorbit. Der Co-Sprühgranulationsprozess wird wie beschrieben durchgeführt ohne jedoch Material am Ausgang der Anlage zu entnehmen. Vielmehr wird das Material komplett über den Mahlventilator in den Prozess zurück geführt bis ein stabile Prozessführung und eine erfindungsgemäße Produktzusammensetzung erreicht ist. Danach beginnt die Einstellung der Kornverteilung mit Produktentnahme wie für einen kontinuierliche Prozessführung beschrieben.

Zur Verdeutlichung der entsprechenden Vorgehensweise wird die Durchführung an dem Beispiel beschrieben, welches zur Herstellung der im folgenden als Produkt E benannten co-gesprühten Zusammensetzung führt. Um ein Ausgangsprodukt zu erhalten welches für die Co-Sprühgranulation eingesetzt werden kann, wird durch eine Vorsprühung ein geeignetes Vorprodukt hergestellt, das in dem Wirbelschichtgranulator für die eigentliche Co-Sprühgranulation, die Hauptsprühung, vorgelegt werden kann:
1. Vorsprühung: In 2,20 kg vollentsalztem Wasser werden bei 20-25°C unter Rühren 0,20 kg Mannit und 0,25 kg Sorbit gelöst. In der klaren Lösung werden anschließend unter Rühren 1,75 kg Calciumhydrogenphosphat Dihydrat suspendiert.
   In einem Wirbelschichtgranulator, wie er beispielsweise in WO 00//76650 A1 beschrieben ist oder wie von der Fa. Glatt vertrieben wird (GPCG 5, Fa.Glatt, Deutschland), werden 0,3 kg Mannitpulver vorgelegt und in Verwirbelung gebracht. Auf dieses Wirbelbett wird die beschriebene Sprühsuspension aufgesprüht.
   Zur Durchführung dieser Co-Sprühgranulation werden zur Steuerung am Wirbelschichtgranulator GPCG 5 folgende Einstellungen durchgeführt:
   Zuluftklappe: ca. 20% (ca. 225m³/h),
   Abluftklappe: ca. 25%
   Zulufttemperatur: ca. 70°C,
   Düse: als Zweistoffdüse 1,2 mm im Top down, obere Düsenstellung, Sprühdruck 3,5 bar;
   Spührate: steigend von 0,02 kg/Minute auf 0,12 kg/Minute;
   Einstellung Ablufttemperatur: ca. 40°C;

   Nach erfolgter Aufsprühung wird das erhaltene Material noch für etwa 10 bis 20 Minuten in der Wirbelschicht nachgetrocknet, wobei die Temperatur der Zuluft so eingestellt wird, dass die Produkttemperatur auf bis zu 50°C steigt.
2. Hauptsprühung: Zu 2,50 kg vollentsalztes Wasser werden unter Rühren 0,50 kg Mannit und 0,25 kg Sorbit zugegeben. Zu der so erhaltenen klaren Lösung werden 1,75 kg Calciumhydrogenphosphat Dihydrat zugegeben. Die entstandene Suspension wird noch etwa eine Stunde nachgerührt, um alle eventuell entstandenen Agglomerate zu zerstören.
   Im Wirbelschichtgranulator (GPCG 5) werden 0,5 kg der Vorsprühung vorgelegt und die Suspension - wie oben anhand der Herstellung der Vorsprühung dargelegt - aufgesprüht.
   An diese erste Hauptsprühung können sich mehrere weitere Sprühungen anschließen, wobei bei jeder Sprühung nur jeweils ein geringer Teil der vorhergehenden Sprühung als Wirbelschichtvorlage eingesetzt wird, z.B. wie oben beschrieben 0,5 kg. Auf diese Weise wird der "nicht co-gesprühte" Anteil im Produkt kontinuierlich reduziert. Die Prüfung der vollständigen Abtrocknung des Produkts erfolgt über die komplexometrische Calcium-Bestimmung (als in-process Prüfung). Gleichzeit kann durch diese Bestimmung auch eine eventuelle "Übertrockung" des Calciumhydrogenphosphat Dihydrats, d.h. ein eventueller Kristallwasserverlust, erkannt werden.

### Versuchsergebnisse

Die durch die verschiedenen Versuche erzielten Ergebnisse sind in den folgenden Tabellen 1 - 5 wiedergegeben.

In Tabelle 1 sind die getesteten Zusammensetzungen mit unterschiedlichen Gewichtsanteilen an Calciumhydrogenphosphat Dihydrat, Mannit und Sorbit zusammengefasst.

Tabelle 2 enthält die ermittelten physikalischen Daten der getesteten Zusammensetzungen. In Tabelle 4 sind die entsprechenden physikalischen Daten für handelsübliche direktverpressbare Calciumhydrogenphosphat Dihydrate und einer tablettierten mechanischen Mischung von DC-Calciumhydrogenphosphat Dihydrat, Mannit und Sorbit zusammengestellt. In Tabelle 3 sind die Tablettierungsdaten, Presskraft, Tablettenhärte, Friabilität, Zerfallszeit, Ausstoßkraft, der getesteten Zusammensetzungen zusammengefasst. In Tabelle 5 sind den entsprechenden Tablettierungsdaten für handelsübliche DC-Calciumhydrogenphosphat Dihydrate diejenigen der besonders bevorzugten cosprühgranulierten Kombinationen von Calciumhydrogenphosphat Dihydrat, Mannit und Sorbit der Beispiele E und D gegenübergestellt.

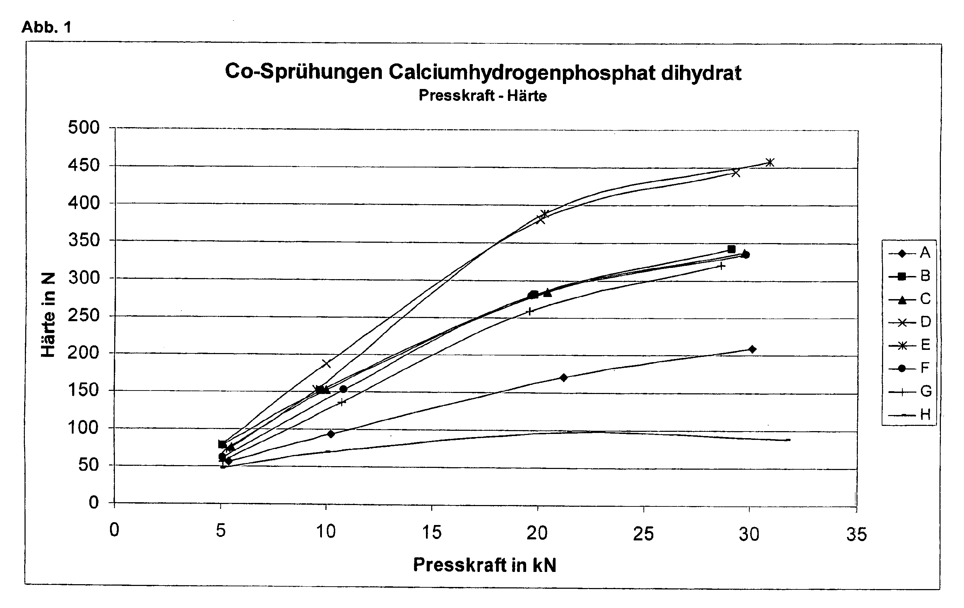

Abb. 1 zeigt die Presskraft Härteprofile für die untersuchten co-gesprühten Calciumhydrogenphosphat Dihydrat-Zusammensetzungen im Vergleich.

Anhand der Profile lässt sich erkennen, dass die Tablettenhärten beim Verpressen von co-sprühgranuliertem Calciumhydrogenphosphat Dihydrat mit geringen Mengen Mannit/Sorbit-Zusatz (Beispiel H) zu Tabletten mit zunehmendem Preßdruck nur zwischen etwa 50 und 100 N variieren. Wird jedoch eine co-gesprühte Zusammensetzung aus 70 Gew.-% Calciumhydrogenphosphat Dihydrat und 30 Gew.-% Mannit unter den gleichen Bedingungen mit steigendem Preßdruck tablettiert, werden Produkte mit Härten zwischen etwa 50 und 220 N erhalten. Für die übrigen untersuchten Zusammensetzungen werden Tabletten mit noch wesentlich höheren Härten unter denselben Bedingungen erhalten. Insbesondere für Zusammensetzungen, in denen das Gew.-Verhältnis von Calciumhydrogenphosphat Dihydrat : Mannit : Sorbit bei 60 : 30 : 10 bzw. 70 : 20 : 10 liegt, werden besonders hohe Härten erzielt.

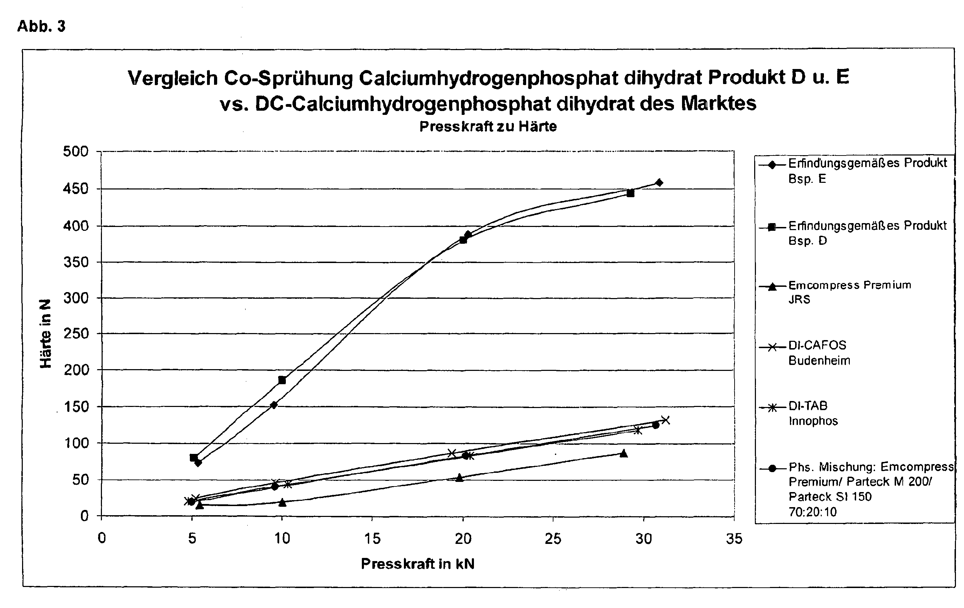

Wie sich in Abb. 3 zeigt, kann durch Vergleich der erfindungsgemäßen Zusammensetzungen mit handelsüblichen direkttablettierfähigen Calciumhydrogenphosphat Dihydrat-Qualitäten festgestellt werden, dass insbesondere für Zusammensetzungen, in denen das Gew.-Verhältnis von Calciumhydrogenphosphat Dihydrat : Mannit: Sorbit bei 60 : 30 : 10 bzw. 70 : 20 : 10 liegt, bei gleichen Presskräften erheblich höhere Tablettenhärten erzielt werden. Dieses gilt aber auch für Zusammensetzungen, in denen Calciumhydrogenphosphat Dihydrat mit mindestens einem der Polyole Mannit oder Sorbit cogesprüht worden ist, wie sich am Vergleich zwischen den Zusammensetzungen A und B und den Reinsubstanzen in den Abbildungen 1 und 3 zeigt. Insbesondere überraschend ist dieses gegenüber physikalischen Mischungen von DC-Calciumhydrogenphosphat Dihydrat, DC-Mannit und DC-Sorbit. Diese zeigen gegenüber den tablettierten Reinsubstanzen bei unterschiedlichen Preßkräften nur vergleichsweise gering erhöhte Tablettenhärten.

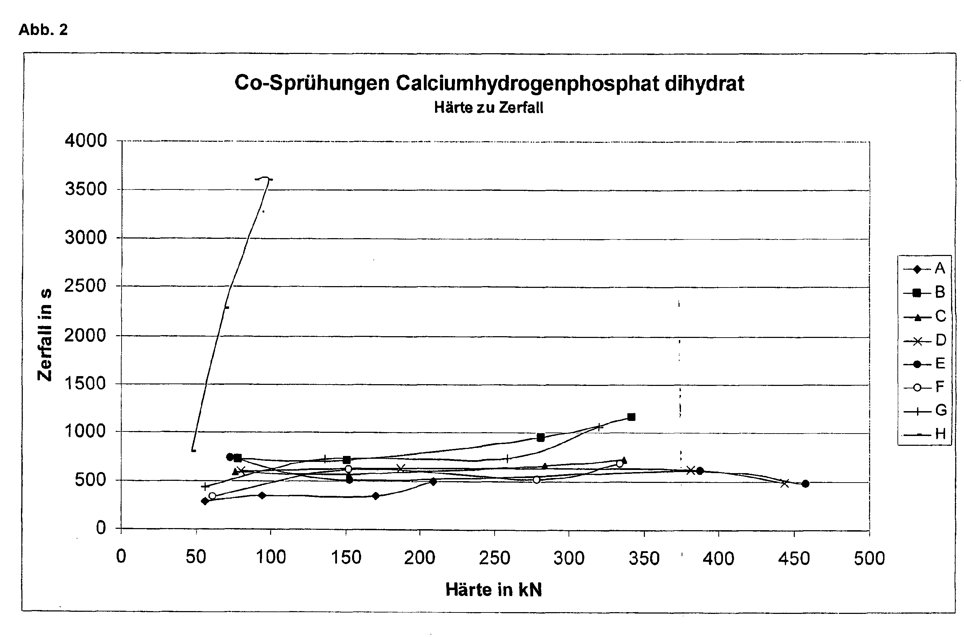

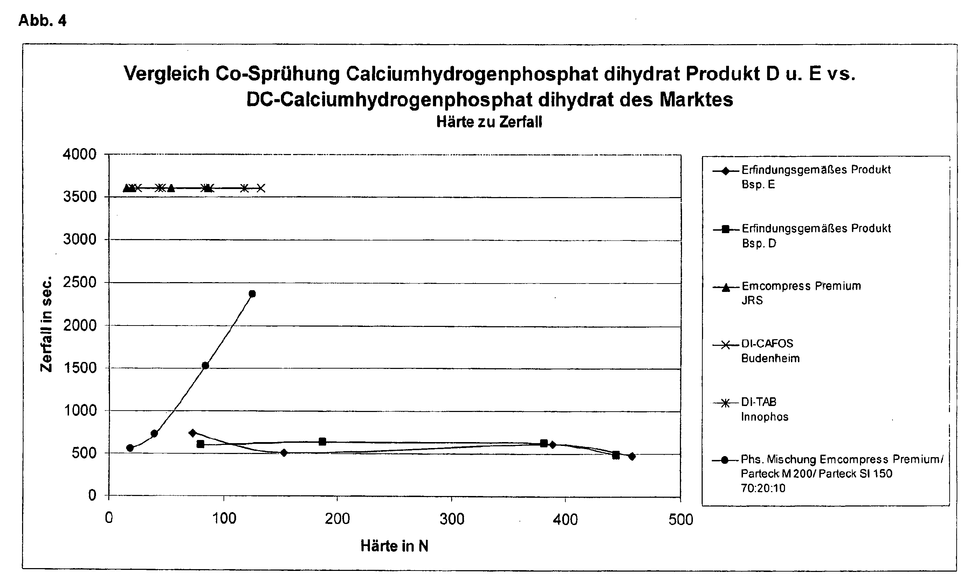

Trotz der erhöhten Tablettenhärten weisen die entsprechenden Tabletten der erfindungsgemäßen Zusammensetzungen sehr kurze Zerfallszeiten im Vergleich zu der Reinsubstanz auf, wie sich aus den Graphischen Darstellungen der Abb. 2 und Abb. 4 sehr gut entnehmen lässt. Während eine tablettierte Zusammensetzung, bestehend aus 90 Gew.% Calciumhydrogenphosphat Dihydrat und jeweils 5 Gew.-% Mannit und Sorbit mit zunehmender Härte zwischen 47 und 89 N eine enorm verlängerte Zerfallszeit von mehr als 3600 sec zeigt, weisen die erfindungsgemäßen Zusammensetzungen trotz zunehmender Härte lediglich Zerfallszeiten im Bereich von etwa 300 sec bis 750 sec auf, abgesehen von Zerfallszeiten von 900 bis 1160 sec für Zusammensetzungen, bestehend aus 70 Gew.-% Calciumhydrogenphosphat Dihydrat und 30 Gew.-% Sorbit bei einer Härte von 342 N und für Zusammensetzungen, bestehend aus 85 Gew.-% Calciumhydrogenphosphat Dihydrat und 10 Gew.-% Mannit und 5 Gew.-% Sorbit.

Während die Zerfallszeiten der Tabletten aus physikalischen Mischungen von DC-Calciumhydrogenphosphat Dihydrat, DC-Mannit und DC-Sorbit mit zunehmender Härte ansteigen, wie aus Abb. 4 zu entnehmen ist, ändern sich die Zerfallszeiten der erfindungsgemäßen Zusammensetzungen mit zunehmenden Härten vergleichsweise wenig, bzw. bleiben bei den besonders bevorzugten Zusammensetzungen D und E nahezu konstant. Zwar bleiben die Zerfallszeiten der Tabletten aus handelsüblichen Calciumhydrogenphosphat Dihydrat-Qualitäten mit zunehmenden Härten auch etwa konstant, jedoch liegen sie grundsätzlich um ein vielfaches höher, d. h. während Tabletten, hergestellt aus erfindungsgemäßen Zusammensetzungen, Zerfallszeiten im Bereich von 300 bis 1160 sec haben, zeigen Tabletten handelsüblicher Calciumhydrogenphosphat Dihydrat-Qualitäten Zerfallszeiten von länger als 3600 sec.

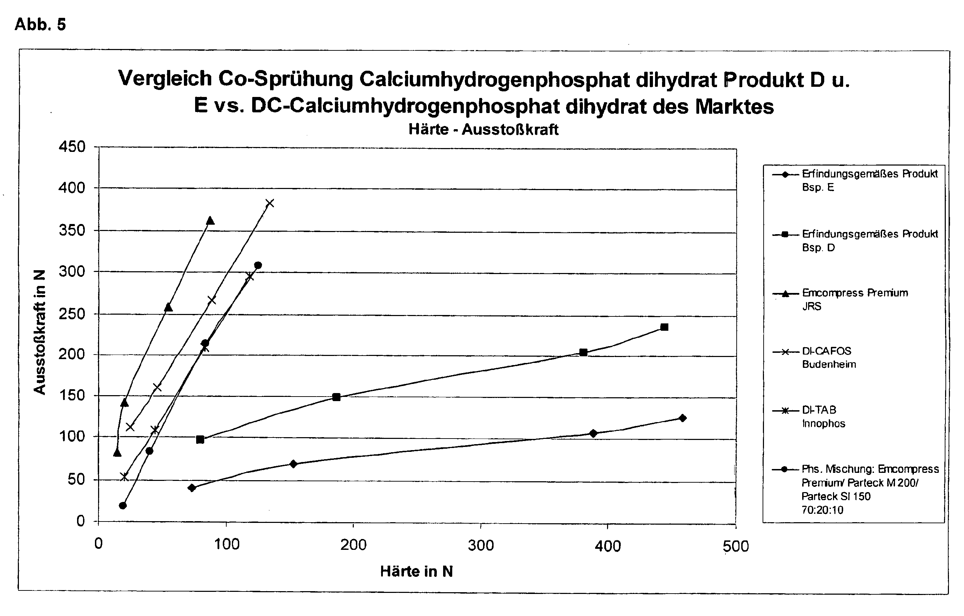

In Abb. 5 sind die Härten der hergestellten Tabletten gegen die Ausstoßungskräfte aufgetragen. Beispielhaft sind dabei die Härten von Tabletten, hergestellt aus Zusammensetzungen der Beispiele D und E und den damit verbundenen Ausstoßungskräften mit denen von entsprechenden handelüblichen Produkten verglichen. Dieser Vergleich zeigt recht anschaulich, dass Tabletten aus erfindungsgemäßen Zusammensetzungen trotz zunehmender Härte mit relativ wenig zunehmenden Ausstoßungskräften aus den Tablettierwerkzeugen ausgestoßen werden können. Die entsprechenden Vergleichsdaten sind auch aus Tabelle 5 zu entnehmen. Im Gegensatz dazu steigen für die verglichenen handelsüblichen Produkte schon bei recht niedriger Zunahme der Tablettenhärte die erforderlichen Austoßungskräfte sehr stark an. Dementsprechend ist die Beanspruchung der Tablettiermaschinen wesentlich geringer bei Verwendung der erfindungsgemäßen direkt verpressbaren Zusammensetzungen im Vergleich zur Verwendung von handelsüblichen Zusammensetzungen.

**Tabelle 1:**

| Produkt | Calciumhydrogenphosphat dihydrat Art. Nr.: 1.2146 Merck KGaA, Deutschland [Gew.-%] | Mannit Art. Nr.: 1.05980 Merck KGaA, Deutschland [Gew.-%] | Sorbit (Parteck SI 400) Art. Nr.: 1.03140 Merck KGaA, Deutschland [Gew.-%] |
|---|---|---|---|
| A *1 | 70 | 30 | |
| B *2 | 70 | | 30 |
| C | 50 | 40 | 10 |
| D | 60 | 30 | 10 |
| E | 70 | 20 | 10 |
| F | 80 | 15 | 5 |
| G | 85 | 10 | 5 |
| H *3 | 90 | 5 | 5 |

| | | | |
|---|---|---|---|
| *1 Vergleichsbeispiel *2 Vergleichsbeispiel *3 Vergeichsbeispiel | | | |

**Tabelle 2:**

| | **A** | **B** | **C** | **D** | **E** | **F** | **G** | **H** |
|---|---|---|---|---|---|---|---|---|
| Schüttdichte [g/ml] | 0,70 | 0,59 | 0,63 | 0,60 | 0,66 | 0,70 | 0,71 | 0,68 |
| Stampfdichte [g/ml] | 1,00 | 0,71 | 0,77 | 0,76 | 0,78 | 0,88 | 0,85 | 0,79 |
| Fließwinkel [°] | 41,3 | 31,8 | 32,6 | 29,1 | 29,6 | 34,7 | 33,1 | 34,7 |
| Hausner Faktor | 1,43 | 1,20 | 1,22 | 1,27 | 1,18 | 1,26 | 1,20 | 1,16 |
| Kompressibilitäts -Index [%] | 30,00 | 16,90 | 18,18 | 21,05 | 15,39 | 20,46 | 16,47 | 13,92 |

| Kornverteilung in [Gew.%] | | | | | | | | |
|---|---|---|---|---|---|---|---|---|
| < 32 µm | 33,63 | 0 | 0 | 0,07 | 0 | 0,09 | 0 | 0,02 |
| 32 - 50 µm | 38,11 | 0,07 | 1,92 | 1,98 | 2,62 | 6,78 | 2,02 | 0,51 |
| 50 - 100 µm | 19,54 | 0,41 | 8,36 | 17,82 | 16,96 | 46,06 | 36,56 | 7,63 |
| 100 - 200 µm | 5,92 | 2,85 | 30,76 | 67,36 | 34,00 | 39,47 | 48,75 | 9,81 |
| 200 - 315 µm | 1,15 | 30,05 | 43,35 | 11,61 | 35,31 | 3,26 | 6,17 | 12,00 |
| 315 - 500 µm | 0,62 | 39,14 | 11,92 | 0,64 | 8,90 | 1,86 | 4,45 | 32,07 |
| 500-710 µm | 0,35 | 27,30 | 2,75 | 0,31 | 2,21 | 0,84 | 1,74 | 22,32 |
| 710 -1000 µm | 0,33 | 0,18 | 0,90 | 0,09 | 0 | 0,92 | 0,31 | 15,35 |
| > 1000 µm | 0,35 | 0 | 0,04 | 0,12 | 0 | 0,72 | 0 | 0,29 |

| Calciumgehalt [%] | | | | | | | | |
|---|---|---|---|---|---|---|---|---|
| - theoretisch | 16,3 | 16,3 | 11,7 | 14,0 | 16,3 | 18,6 | 19,8 | 21,0 |
| - gefunden | 16,7 | 16,8 | 12,0 | 14,0 | 16,5 | 18,7 | 20,0 | 20,8 |

**Tabelle 3:**

| Co-gesprühtes Produkt | Preßkraft [kN] | | Tablettenhärte [N] | Friabilität [%] | Zerfallszeit [sec] | Ausstoßkraft [N] |
|---|---|---|---|---|---|---|
| | soll | ist | | | | |
| A | 5 | 5,4 | 56 | 1,05 | 286 | 75 |
| | 10 | 10,2 | 94 | 0,335 | 341 | 140 |
| | 20 | 21,2 | 170 | 0,172 | 351 | 292 |
| | 30 | 30,1 | 209 | 0,145 | 497 | 393 |
| B | 5 | 5,1 | 78 | 0,059 | 725 | 76 |
| | 10 | 9,7 | 151 | 0,264 | 717 | 119 |
| | 20 | 19,8 | 281 | 0,103 | 948 | 179 |
| | 30 | 29,1 | 342 | 0,103 | 1158 | 200 |
| C | 5 | 5,5 | 76 | 0,106 | 589 | 74 |
| | 10 | 10,0 | 152 | 0,066 | 566 | 118 |
| | 20 | 20,4 | 284 | 0,084 | 662 | 175 |
| | 30 | 29,7 | 337 | 0,093 | 727 | 208 |
| D | 5 | 5,1 | 80 | 0,245 | 602 | 97 |
| | 10 | 10,0 | 187 | 0,132 | 631 | 148 |
| | 20 | 20,1 | 381 | 0,111 | 623 | 205 |
| | 30 | 29,3 | 444 | 0,129 | 490 | 236 |
| E | 5 | 5,3 | 73 | 0,019 | 732 | 40 |
| | 10 | 9,5 | 153 | 0,014 | 505 | 69 |
| | 20 | 20,3 | 388 | 0,059 | 613 | 107 |
| | 30 | 30,9 | 458 | 0,080 | 474 | 125 |
| F | 5 | 5,1 | 61 | 0,129 | 336 | 63 |
| | 10 | 10,8 | 152 | 0,103 | 621 | 108 |
| | 20 | 19,7 | 279 | 0,114 | 523 | 160 |
| | 30 | 29,8 | 334 | 0,118 | 687 | 201 |
| G | 5 | 5,1 | 56 | 0,133 | 434 | 49 |
| | 10 | 10,7 | 136 | 0,108 | 726 | 95 |
| | 20 | 19,6 | 259 | 0,100 | 734 | 155 |
| | 30 | 28,6 | 320 | 0,102 | 1059 | 199 |
| H | 5 | 5,0 | 47 | 0,173 | 794 | 51 |
| | 10 | 10,0 | 69 | 0,150 | 2278 | 87 |
| | 20 | 21,4 | 98 | 0,149 | >3600 | 156 |
| | 30 | 31,7 | 89 | 0,118 | >3600 | 165 |

**Tabelle 4:**

| | Emcompress Premium | DI-CAFOS | DI-TAB | Emcompress Premium/ Parteck M 200/ Parteck SI 150 70:20:10 mech. Misch. |
|---|---|---|---|---|
| Schüttdichte [g/ml] | 0,72 | 0,84 | 0,82 | 0,70 |
| Stampfdichte [g/ml] | 0,90 | 1,02 | 1,03 | 0,86 |
| Fließwinkel [°] | 31,8 | 29,6 | 30,1 | 30,6 |
| Hausner Faktor | 1,25 | 1,21 | 1,26 | 1,23 |
| Kompressibilitäts-Index [%] | 20,00 | 17,65 | 20,39 | 18,61 |

| Kornverteilung in [Gew.%] | | | | |
|---|---|---|---|---|
| < 32 µm | 0,02 | 0,10 | 0,02 | 0 |
| 32 - 50 µm | 1,62 | 1,29 | 1,79 | 1,54 |
| 50 - 100 µm | 15,64 | 9,76 | 13,35 | 19,50 |
| 100 - 200 µm | 67,62 | 72,14 | 41,45 | 60,24 |
| 200 - 315 µm | 14,71 | 16,48 | 42,93 | 17,14 |
| 315 - 500 µm | 0,32 | 0,23 | 0,42 | 1,32 |
| 500 - 710 µm | 0,05 | 0 | 0,02 | 0,16 |
| 710 - 1000 µm | 0,02 | 0 | 0,02 | 0,05 |
| > 1000 µm | 0 | 0 | 0 | 0,05 |

**Tabelle 5:**

| Produkt | Presskraft [kN] | | Tablettenhärte [N] | Friabilität [%] | Zerfallszeit [sec.] | Ausstoßkraft [N] |
|---|---|---|---|---|---|---|
| | soll | ist | | | | |
| Erfindungsgemäßes Produkt Bsp. E | 5 | 5,3 | 73 | 0,019 | 732 | 40 |
| | 10 | 9,5 | 153 | 0,014 | 505 | 69 |
| | 20 | 20,3 | 388 | 0,059 | 613 | 107 |
| | 30 | 30,9 | 458 | 0,080 | 474 | 125 |
| Erfindungsgemäßes Produkt Bsp. D | 5 | 5,1 | 80 | 0,245 | 602 | 97 |
| | 10 | 10 | 187 | 0,132 | 631 | 148 |
| | 20 | 20,1 | 381 | 0,111 | 623 | 205 |
| | 30 | 29,3 | 444 | 0,129 | 490 | 236 |
| Emcompress Premium JRS * | 5 | 5,4 | 15 | 100 | >3600 | 82 |
| | 10 | 10,0 | 20 | 90,239 | >3600 | 141 |
| | 20 | 19,8 | 54 | 0,632 | >3600 | 259 |
| | 30 | 28,9 | 87 | 0,350 | >3600 | 362 |
| DI-CAFOS Budenheim * | 5 | 5,2 | 25 | 31,07 | >3600 | 112 |
| | 10 | 9,6 | 46 | 0,507 | >3600 | 160 |
| | 20 | 19,4 | 88 | 0,293 | >3600 | 267 |
| | 30 | 31,2 | 133 | 0,188 | >3600 | 383 |
| DI-TAB Innophos * | 5 | 4,8 | 20 | 100 | >3600 | 53 |
| | 10 | 10,3 | 44 | 0,612 | >3600 | 108 |
| | 20 | 20,4 | 83 | 0,279 | >3600 | 209 |
| | 30 | 29,7 | 118 | 0,192 | >3600 | 296 |
| Physikalische | 5 | 5,0 | 19 | 4,282 | 555 | 18 |
| Mischung * | 10 | 9,6 | 40 | 1,223 | 725 | 83 |
| Emcompress | 20 | 20,2 | 84 | 0,474 | 1517 | 214 |
| Premium/ Parteck M 200/ Parteck SI 150, 70:20:10 | 30 | 30,7 | 125 | 0,174 | 2366 | 309 |

| | | | | | | |
|---|---|---|---|---|---|---|
| * Vergleichsbeispiel | | | | | | |

## Patentansprüche

1. Direkt verpressbare Zusammensetzung für die Tablettenherstellung, **dadurch gekennzeichnet, dass** sie Calciumhydrogenphosphat Dihydrat : Mannit : Sorbit in einem Gewichtsverhältnis im Bereich zwischen 50 : 40 : 10 und 85 : 10 : 5, enthält, welche erhältlich ist durch ein Verfahren, worin
eine Lösung bzw. Suspension, enthaltend Calciumhydrogenphosphat Dihydrat, Mannit und Sorbit in einem Gewichtsverhältnis zueinander im Bereich von 50 : 40 : 10 bis 85 : 10 : 5 in Wasser, wobei in 4 Teilen Wasser 4 Teile Feststoff gelöst, bzw. suspendiert werden, und entweder chargenweise oder kontinuierlich in einem Wirbelschichtgranulator einem Co-Sprühgranulationsprozess unterworfen werden.

2. Direkt verpressbare Zusammensetzung nach Anspruch 1 für die Tablettenherstellung, **dadurch gekennzeichnet, dass** sie aus einer Kombination von Calciumhydrogenphosphat Dihydrat, Mannit und Sorbit in einem Gewichtsverhältnis im Bereich von 60 : 30 : 10 und 70 : 20 : 10 besteht.

3. Direkt verpressbare Zusammensetzung nach einem der vorhergehenden Ansprüche 1 oder 2, **dadurch gekennzeichnet, dass** sie einen Fließwinkel im Bereich von 29 bis 42°, insbesondere von 29 bis 35°, besonders bevorzugt im Bereich von 29 bis 30°, aufweist.

4. Direkt verpressbare Zusammensetzung nach einem oder mehreren der vorhergehenden Ansprüche 1 bis 3, **dadurch gekennzeichnet, dass** sie eine Schüttdichte im Bereich von 0,55 - 0,75 g/ml aufweist bei einer Stampfdichte im Bereich von 0,73 - 0,90 g/ml.

5. Direkt verpressbare Zusammensetzung nach einem oder mehreren der vorhergehenden Ansprüche 1 bis 4, **dadurch gekennzeichnet, dass** sie eine Kornverteilung aufweist von 8 bis 47 Gew.-% im Bereich von 50 bis 100 µm, 30 bis 68 Gew.-% im Bereich 100 bis 200 µm, 6 bis 44 Gew.-% im Bereich von 200 bis 315 µm und 0,6 bis 12 Gew.-% im Bereich von 315 - 500µm, wobei der Gewichtsanteil der Kornfraktion <50 µm nicht mehr als 7 Gew.-% und der Gewichtsanteil der Kornfraktion >500µm nicht mehr als 4 Gew.-% beträgt.

6. Direkt verpressbare Zusammensetzung nach einem oder mehreren der vorhergehenden Ansprüche 1 bis 5, **dadurch gekennzeichnet, dass** sie einen Calciumgehalt im Bereich von 11 bis 20 Gew.-% aufweist.

7. Direkt verpressbare Zusammensetzung nach einem oder mehreren der vorhergehenden Ansprüche 1 bis 6, **dadurch gekennzeichnet, dass** sie nach Kompression mit einer Preßkraft von 20 kN Tabletten mit Härten von > 250 N liefert, verbunden mit einer Ausstoßkraft <210 N, einer Friabilität <0,12 %, einer Zerfallszeit <735 Sekunden.

8. Direkt verpressbare Zusammensetzung nach einem oder mehreren der vorhergehenden Ansprüche 1 bis 6, **dadurch gekennzeichnet, dass** sie nach Kompression mit einer Preßkraft von 20 kN Preßlinge mit Härten von >300N liefert, verbunden mit einer Ausstoßkraft von <110 N, einer Friabilität von <0,06 % und einer Zerfallszeit von <620 Sekunden.

9. Direkt verpressbare Zusammensetzung nach einem oder mehreren der vorhergehenden Ansprüche 1 bis 6, **dadurch gekennzeichnet, dass** sie nach Kompression mit einer Preßkraft von 30 kN Preßlinge mit Härten von >430N liefert, verbunden mit einer Ausstoßkraft von <130N, einer Friabilität von maximal 0,08 % und einer Zerfallszeit von <480 Sekunden.

10. Zusammensetzung bzw. Formulierung, **dadurch gekennzeichnet, dass** sie eine direkt verpressbare Zusammensetzung nach einem oder mehreren der vorhergehenden Ansprüche 1 bis 9 enthalten und als feste Form oder Komprimat vorliegen.

11. Zusammensetzung bzw. Formulierung nach Anspruch 10, **dadurch gekennzeichnet, dass** sie einen oder mehrere nicht wasserlösliche und/oder wasserlösliche Zusätze homogen verteilt enthält.

12. Zusammensetzung bzw. Formulierung nach Anspruch 10 oder 11, **dadurch gekennzeichnet, dass** sie einen oder mehrere Zusätze, ausgewählt aus der Gruppe pharmazeutische Wirkstoffe, Pflanzenextrakte, Süßstoffe, Farbstoffe, Zitronensäure, Vitamine und Spurenelemente enthält.

13. Zusammensetzung bzw. Formulierung nach einem der Ansprüche 10, 11 oder 12, **dadurch gekennzeichnet, dass** sie einen oder mehrere pharmazeutische Wirkstoffe aus der Gruppe der Analgetica enthält.

14. Zusammensetzung bzw. Formulierung nach einem der Ansprüche 10, 11, 12 oder 13, **dadurch gekennzeichnet, dass** sie einen oder mehrere Süßstoffe, ausgewählt aus der Gruppe Acesulfam K, Aspartarm, Saccharin, Cyclamat, Sucralose und Neohesperidin DC enthält.

## Claims

1. Directly compressible composition for the production of tablets, **characterised in that** it comprises calcium hydrogenphosphate dihydrate : mannitol : sorbitol in a weight ratio in the range between 50 : 40 : 10 and 85 : 10 : 5,
which is obtainable by a process in which
a solution or suspension comprising calcium hydrogenphosphate dihydrate, mannitol and sorbitol in a weight ratio to one another in the range from 50 : 40 : 10 to 85 : 10 : 5 in water, where 4 parts of solid are dissolved or suspended in 4 parts of water, is subjected to a co-spray-granulation process, either batchwise or continuously in a fluidised-bed granulator.

2. Directly compressible composition according to Claim 1 for the production of tablets, **characterised in that** it consists of a combination of calcium hydrogenphosphate dihydrate, mannitol and sorbitol in a weight ratio in the range from 60 : 30 : 10 to 70 : 20 : 10.

3. Directly compressible composition according to one of the preceding Claims 1 or 2, **characterised in that** it has a flow angle in the range from 29 to 42°, in particular from 29 to 35°, particularly preferably in the range from 29 to 30°.

4. Directly compressible composition according to one or more of the preceding Claims 1 to 3, **characterised in that** it has a bulk density in the range 0.55 - 0.75 g/ml with a tapped density in the range 0.73 - 0.90 g/ml.

5. Directly compressible composition according to one or more of the preceding Claims 1 to 4, **characterised in that** it has a particle-size distribution of 8 to 47% by weight in the range from 50 to 100 µm, 30 to 68% by weight in the range 100 to 200 µm, 6 to 44% by weight in the range from 200 to 315 µm and 0.6 to 12% by weight in the range 315 - 500 µm, where the proportion by weight of the particle-size fraction < 50 µm is not more than 7% by weight and the proportion by weight of the particle-size fraction > 500 µm is not more than 4% by weight.

6. Directly compressible composition according to one or more of the preceding Claims 1 to 5, **characterised in that** it has a calcium content in the range from 11 to 20% by weight.

7. Directly compressible composition according to one or more of the preceding Claims 1 to 6, **characterised in that** it gives, after compression with a pressing force of 20 kN, tablets having hardnesses of > 250 N, together with an ejection force of < 210 N, a friability of < 0.12%, a disintegration time of < 735 seconds.

8. Directly compressible composition according to one or more of the preceding Claims 1 to 6, **characterised in that** it gives, after compression with a pressing force of 20 kN, pressed tablets having hardnesses of > 300 N, together with an ejection force of < 110 N, a friability of < 0.06% and a disintegration time of < 620 seconds.

9. Directly compressible composition according to one or more of the preceding Claims 1 to 6, **characterised in that** it gives, after compression with a pressing force of 30 kN, pressed tablets having hardnesses of > 430 N, together with an ejection force of < 130 N, a friability of at most 0.08% and a disintegration time of < 480 seconds.

10. Composition or formulation, **characterised in that** it comprises a directly compressible composition according to one or more of the preceding Claims 1 to 9 and is in solid form or in the form of a compressate.

11. Composition or formulation according to Claim 10, **characterised in that** it comprises one or more homogeneously distributed, water-insoluble and/or water-soluble additives.

12. Composition or formulation according to Claim 10 or 11, **characterised in that** it comprises one or more additives selected from the group pharmaceutical active compounds, plant extracts, sweeteners, dyes, citric acid, vitamins and trace elements.

13. Composition or formulation according to one of Claims 10, 11 or 12, **characterised in that** it comprises one or more pharmaceutical active compounds from the group of the analgesics.

14. Composition or formulation according to one of Claims 10, 11, 12 or 13, **characterised in that** it comprises one or more sweeteners selected from the group acesulfame K, aspartame, saccharin, cyclamate, sucralose and neohesperidin DC.

## Revendications

1. Composition directement compressible pour la fabrication de comprimés, **caractérisée en ce qu'**elle comprend dihydrate d'hydrogène-phosphate de calcium : mannitol : sorbitol selon un rapport en poids dans la plage entre 50 : 40 : 10 et 85 : 10 : 5,
laquelle peut être obtenue au moyen d'un processus selon lequel une solution ou une suspension comprenant du dihydrate d'hydrogène-phosphate de calcium, du mannitol et du sorbitol selon un rapport en poids les uns par rapport aux autres dans la plage de 50 : 40 : 10 à 85 : 10 : 5 dans de l'eau, où 4 parties de solide sont dissoutes ou mises en suspension dans 4 parties d'eau, est soumise à un processus de co-pulvérisation-granulation, soit par lots, soit en continu dans un granulateur à lit fluidisé.

2. Composition directement compressible selon la revendication 1 pour la fabrication de comprimés, **caractérisée en ce qu'**elle est constituée par une combinaison de dihydrate d'hydrogènephosphate de calcium, de mannitol et de sorbitol selon un rapport en poids dans la plage de 60 : 30: 10 à 70: 20: 10.

3. Composition directement compressible selon l'une des revendications précédentes 1 ou 2, **caractérisée en ce qu'**elle présente un angle de fluage dans la plage de 29 à 42°, en particulier de 29 à 35°, de façon particulièrement préférable, dans la plage de 29 à 30°.

4. Composition directement compressible selon une ou plusieurs des revendications précédentes 1 à 3, **caractérisée en ce qu'**elle présente une densité dans la masse dans la plage 0,55 - 0,75 g/ml avec une densité après tassement dans la plage 0,73 - 0,90 g/ml.

5. Composition directement compressible selon une ou plusieurs des revendications précédentes 1 à 4, **caractérisée en ce qu'**elle présente une distribution de dimensions de particule de 8 à 47% en poids dans la plage de 50 à 100 µm, de 30 à 68% en poids dans la plage de 100 à 200 µm, de 6 à 44% en poids dans la plage de 200 à 315 µm et de 0,6 à 12% en poids dans la plage de 315 - 500 µm, où la proportion en poids de la fraction de dimensions de particule < 50 µm n'est pas supérieure à 7% en poids et la proportion en poids de la fraction de dimensions de particule > 500 µm n'est pas supérieure à 4% en poids.

6. Composition directement compressible selon une ou plusieurs des revendications précédentes 1 à 5, **caractérisée en ce qu'**elle présente une teneur en calcium dans la plage de 11 à 20% en poids.

7. Composition directement compressible selon une ou plusieurs des revendications précédentes 1 à 6, **caractérisée en ce qu'**elle permet d'obtenir, après compression selon une force de pression de 20 kN, des comprimés présentant des duretés > 250 N, en association avec une force d'éjection < 210 N, une friabilité < 0,12%, un temps de désintégration < 735 secondes.

8. Composition directement compressible selon une ou plusieurs des revendications précédentes 1 à 6, **caractérisée en ce qu'**elle permet d'obtenir, après compression selon une force de pression de 20 kN, des comprimés pressés présentant des duretés > 300 N, en association avec une force d'éjection < 110 N, une friabilité < 0,06% et un temps de désintégration < 620 secondes.

9. Composition directement compressible selon une ou plusieurs des revendications précédentes 1 à 6, **caractérisée en ce qu'**elle permet d'obtenir, après compression selon une force de pression de 30 kN, des comprimés pressés présentant des duretés > 430 N, en association avec une force d'éjection < 130 N, une friabilité d'au plus 0,08% et un temps de désintégration < 480 secondes.

10. Composition ou formulation, **caractérisée en ce qu'**elle comprend une composition directement compressible selon une ou plusieurs des revendications précédentes 1 à 9 et est sous forme solide ou sous la forme d'une matière comprimée.

11. Composition ou formulation selon la revendication 10, **caractérisée en ce qu'**elle comprend un ou plusieurs additifs distribués de façon homogène, non solubles à l'eau et/ou solubles à l'eau.

12. Composition ou formulation selon la revendication 10 ou 11, **caractérisée en ce qu'**elle comprend un ou plusieurs additifs choisis parmi le groupe constitué par des composés pharmaceutiquement actifs, des extraits de plantes, des édulcorants, des colorants, de l'acide citrique, des vitamines et des éléments trace.

13. Composition ou formulation selon l'une des revendications 10, 11 ou 12, **caractérisée en ce qu'**elle comprend un ou plusieurs composés pharmaceutiquement actifs pris parmi le groupe des analgésiques.

14. Composition ou formulation selon l'une des revendications 10, 11, 12 ou 13, **caractérisée en ce qu'**elle comprend un ou plusieurs édulcorants choisis parmi le groupe constitué par acésulfame K, aspartame, saccharine, cyclamate, sucralose et néohespéridine DC.
